# EUROPEAN PATENT APPLICATION

(11) **EP 4 311 556 A1**
(43) Date of publication of application: **31.01.2024**
(21) Application number: 22382725.4
(22) Date of filing: 28.07.2022
(51) Int. Cl.: A61K 38/57, C07K 14/81, A61P 7/02, A61P 9/10

(54) **ANTITHROMBIN IN STROKE**

(71) Applicant: Grifols Worldwide Operations Limited, Dublin 22 (IE)
(72) Inventor: HORRILLO SAURA, RAQUEL, 08150 PARETS DEL VALLES (ES); COSTA RIEROLA, MONTSERRAT, 08150 PARETS DEL VALLES (ES)
(74) Representative: ABG Intellectual Property Law, S.L.

(57) **Abstract**

The present invention relates to the use of Antithrombin in the treatment of stroke.

## Description

### Field of the Invention

The present invention relates to the use of Antithrombin in the treatment of stroke.

### Background

Stroke is one of the most prevalent categories of non-traumatic brain injury and occurs when the blood supply to part of the brain is interrupted or reduced, preventing brain tissue from getting oxygen and nutrients. Neurodegenerative processes commence almost immediately, and brain cells begin to die in minutes. An ischemic stroke occurs when a blood vessel (artery) supplying blood to an area of the brain becomes blocked by a blood clot. A haemorrhagic stroke happens when an artery in the brain leaks or ruptures. Of the two, ischemic stroke is the most common form of stroke.

The pervasiveness of stroke in modern society produces an immense burden on health care infrastructure and spend. At present, recombinant tissue plasminogen activator (rtPA) is the only FDA-approved therapeutic agent for ischemic stroke. The major functions of rtPA are dissolving blood clots and promoting reperfusion. An alternative method for re-establishing a blocked blood flow is by a surgical intervention.

rtPA treatment has a narrow therapeutic time-window and as such its widespread applicability is limited. Moreover, although restoring perfusion via rtPA to the ischemic tissue is important, cascades of necrosis, apoptosis, and inflammation commence within minutes of severe oxygen deprivation. Increasingly, it is posited that genetically programmed neural cell death during post-ischemic tissue inflammation (which can last days to weeks) contributes significantly to the ultimate pathology because of delays in patient treatment/evaluation. As such, permanent neural and neuronal damage can arise before a patient even presents for evaluation.

There are many reports of molecules in the prior art that exert a neuroprotective effect so as to limit the damage caused by disorders such as strokes, and traumatic injuries to the brain and spinal cord. One such example is U.S. Patent Publication No. 2016008437 in the name of Grifols Worldwide Operations Ltd which discloses apo-transferrin as exerting a neuroprotective effect by modulating the activity of Hypoxia Inducible Factors (HIF) in a rat model of stroke. The inventors observed a neuroprotective effect for apo-transferrin manifesting in a decrease in the volume of the infarcted area in the rats treated with apo-transferrin when compared with control rats.

It is immediately apparent that the severe and debilitating effects of neurodegeneration associated with brain injury warrant considerable focus and attention. As such, alternative therapies focussing on this need are highly desirable.

### Description of the Invention

The words "comprises/comprising" and the words "having/including" when used herein with reference to the present invention are used to specify the presence of stated features, integers, steps or components but do not preclude the presence or addition of one or more other features, integers, steps, components or groups thereof.

It should be appreciated by those skilled in the art that the specific embodiments disclosed herein should not be read in isolation, and that the present specification intends for the disclosed embodiments to be read in combination with one another as opposed to individually. As such, each embodiment may serve as a basis for modifying or limiting other embodiments disclosed herein.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "10 to 100" should be interpreted to include not only the explicitly recited values of 10 to 100, but also include individual value and sub-ranges within the indicated range. Thus, included in this numerical range are individual values such as 10, 11, 12, 13... 97, 98, 99, 100 and sub-ranges such as from 10 to 40, from 25 to 40 and 50 to 60, *etc.* This same principle applies to ranges reciting only one numerical value, such as "at least 10". Furthermore, such an interpretation should apply regardless of the breadth of the range or the characteristics being described.

### Treatment of the Invention

In a first aspect, the present invention provides for a pharmaceutical composition comprising antithrombin for use in the treatment of a stroke in a patient in need thereof, wherein the antithrombin is administered at a dose sufficient to:
increase the patient's plasmatic antithrombin activity to greater than about 120 %; or
increase the patient's plasmatic antithrombin concentration to greater than about 1.2 IU/mL.

In one embodiment, the patient has suffered an ischemic stroke. For example, the patient may have suffered a focal brain ischemic stroke.

In some embodiments, the antithrombin may be administered at a dose sufficient to increase the patient's plasmatic antithrombin activity to greater than about 120 %. For example, the antithrombin may be administered at a dose sufficient to increase the patient's plasmatic antithrombin activity to greater than about 120 % but less than about 200 %. For example, the antithrombin may be administered at a dose sufficient to increase the patient's plasmatic antithrombin activity to greater than about 130 %. For example, the antithrombin may be administered at a dose sufficient to increase the patient's plasmatic antithrombin activity to greater than about 130 % but less than about 200 %. For example, the antithrombin may be administered at a dose sufficient to increase the patient's plasmatic antithrombin activity to greater than about 140 %. For example, the antithrombin may be administered at a dose sufficient to increase the patient's plasmatic antithrombin activity to greater than about 140 % but less than about 200 %. For example, the antithrombin may be administered at a dose sufficient to increase the patient's plasmatic antithrombin activity to greater than about 150 %. For example, the antithrombin may be administered at a dose sufficient to increase the patient's plasmatic antithrombin activity to greater than about 150 % but less than about 200 %. For example, the antithrombin may be administered at a dose sufficient to increase the patient's plasmatic antithrombin activity to greater than about 160 %. For example, the antithrombin may be administered at a dose sufficient to increase the patient's plasmatic antithrombin activity to greater than about 160 % but less than about 200 %.

Antithrombin activity is measured using standard techniques that are common general knowledge to those of skill in the art. For example, patient plasma may be mixed with heparin and thrombin and the extent of thrombin inhibition (%) is proportional to the functional antithrombin activity of the patient sample. Thrombin activity is measured by the rate of conversion of a chromogenic substrate and the signal obtained (if any) used to calculate antithrombin activity.

In other embodiments, the antithrombin may be administered at a dose sufficient to increase the patient's plasmatic antithrombin concentration to greater than about 1.2 IU/mL. For example, the antithrombin may be administered at a dose sufficient to increase the patient's plasmatic antithrombin concentration to greater than about 1.2 lU/mL but less than about 2.0 IU/mL. For example, the antithrombin may be administered at a dose sufficient to increase the patient's plasmatic antithrombin concentration to greater than about 1.3 IU/mL. For example, the antithrombin may be administered at a dose sufficient to increase the patient's plasmatic antithrombin concentration to greater than about 1.3 lU/mL but less than about 2.0 IU/mL. For example, the antithrombin may be administered at a dose sufficient to increase the patient's plasmatic antithrombin concentration to greater than about 1.4 IU/mL. For example, the antithrombin may be administered at a dose sufficient to increase the patient's plasmatic antithrombin concentration to greater than about 1.4 lU/mL but less than about 2.0 IU/mL. For example, the antithrombin may be administered at a dose sufficient to increase the patient's plasmatic antithrombin concentration to greater than about 1.5 IU/mL. For example, the antithrombin may be administered at a dose sufficient to increase the patient's plasmatic antithrombin concentration to greater than about 1.5 lU/mL but less than about 2.0 IU/mL. For example, the antithrombin may be administered at a dose sufficient to increase the patient's plasmatic antithrombin concentration to greater than about 1.6 IU/mL. For example, the antithrombin may be administered at a dose sufficient to increase the patient's plasmatic antithrombin concentration to greater than about 1.6 lU/mL but less than about 2.0 IU/mL.

Antithrombin concentration can be measured using standard techniques that are common general knowledge to those of skill in the art. For example, patient plasma may be contacted with an antibody specific to antithrombin as part of a traditional chromogenic immunoassay or ELISA, and the antithrombin concentration determined against control samples of known concentration.

In certain embodiments, the antithrombin may be administered at a dose sufficient to increase the patient's plasmatic antithrombin levels to greater than about 140 µg/mL. For example, the antithrombin may be administered at a dose sufficient to increase the patient's plasmatic antithrombin levels to greater than about 140 µg/mL but less than about 267 µg/mL. For example, the antithrombin may be administered at a dose sufficient to increase the patient's plasmatic antithrombin levels to greater than about 160 µg/mL. For example, the antithrombin may be administered at a dose sufficient to increase the patient's plasmatic antithrombin levels to greater than about 160 µg/mL but less than about 267 µg/mL. For example, the antithrombin may be administered at a dose sufficient to increase the patient's plasmatic antithrombin levels to greater than about 180 µg/mL. For example, the antithrombin may be administered at a dose sufficient to increase the patient's plasmatic antithrombin levels to greater than about 180 µg/mL but less than about 267 µg/mL. For example, the antithrombin may be administered at a dose sufficient to increase the patient's plasmatic antithrombin levels to greater than about 200 µg/mL. For example, the antithrombin may be administered at a dose sufficient to increase the patient's plasmatic antithrombin levels to greater than about 200 µg/mL but less than about 267 µg/mL. For example, the antithrombin may be administered at a dose sufficient to increase the patient's plasmatic antithrombin levels to greater than about 220 µg/mL. For example, the antithrombin may be administered at a dose sufficient to increase the patient's plasmatic antithrombin levels to greater than about 220 µg/mL but less than about 267 µg/mL.

In certain embodiments, the antithrombin may be administered to the patient in multiple doses.

Advantageously, the compositions of the present invention administer antithrombin to patients at concentrations effective to mitigate the effects of stroke without an attendant increase in bleeding risk that can be associated with AT supplementation.

It should be appreciated by those skilled in the art that the specific embodiments disclosed within paragraphs [0010] - [0018] should not be read in isolation, and that the present specification intends for these embodiments to be disclosed in combination with other embodiments as opposed to being disclosed individually.

### Antithrombin

Antithrombin is a single-chain glycoprotein (68 kD) plasma-based serine protease inhibitor having a principle role in maintaining balance in the coagulation system predominantly by inactivating a number of coagulation factors, such as FXa. Moreover, enhancement of antithrombin activity is the mechanism by which heparin achieves its anticoagulant effect. In addition to its role as an anticoagulant, antithrombin has anti-inflammatory properties. Amongst its many names, antithrombin is also commonly termed antithrombin III, AT, or ATIII; all of which are applicable in the present specification.

The present invention includes all wild type mammalian, and functionally active mutants of antithrombin within its scope. The present invention further includes within its scope both cleaved and latent forms of wild type antithrombin. Particularly preferred is human antithrombin, for example both latent and cleaved forms of human antithrombin. For example, human antithrombin (UniProtKB Seq. No. P01008) having the amino acid sequence set forth in SEQ ID NO: 1.

Antithrombin utilised in the present invention may be plasma-derived or recombinant. Plasma-derived antithrombin is purified from the blood plasma of blood donors by processes well known to those of skill in the art. Suitable commercial preparations of human plasma-derived antithrombin illustrative of materials useful in the present invention include, without limitation, a preparation sold under the brand name THROMBATE III.

Illustrative recombinant forms of antithrombin useful in the present invention include, without limitation, AT alfa (ATryn^{®}) and AT gamma.

The present specification includes within its scope recombinant derivatives of antithrombin that differ from the wild type amino acid sequence of the human protein outlined in SEQ ID NO: 1, by one or more substitutions, one or more deletions, or one or more insertions that may not materially alter the structure, or hydropathic nature of the recombinant protein relative to the wild type protein. Recombinant variants of antithrombin within the scope of the present invention may additionally comprise at least one post translational modification, such as pegylation, glycosylation, polysialylation, or combinations thereof.

In one embodiment, the present invention contemplates recombinant variants of antithrombin having one or more conservative substitutions relative to the wild type protein in SEQ ID NO: 1. A "conservative substitution" is one in which an amino acid is substituted for another amino acid that has similar properties, such that one skilled in the art of peptide chemistry would expect the secondary structure and hydropathic nature of the polypeptide to be substantially unchanged. Generally, change within the following groups of amino acids represent conservative changes: (1) ala, pro, gly, glu, asp, gin, asn, ser, thr; (2) cys, ser, tyr, thr; (3) val, ile, leu, met, ala, phe; (4) lys, arg, his; and (5) phe, tyr, trp, his.

For example, the recombinant antithrombin within the scope of the present invention may possess at least 90 %, 95 %, 96 %, 97 %, 98 % or 99 % homology with the wild type human antithrombin protein outlined in SEQ ID NO: 1.

The skilled person will appreciate that recombinant proteins can be obtained utilising standard techniques well known in the art of protein expression, production and purification. Nucleic acid sequences of a recombinant protein of interest can be inserted in any expression vector suitable for expression in the elected host cell, e.g. mammalian cells, insect cells, plant cells, yeast, and bacteria.

As used herein, the term "expression vector" refers to an entity capable of introducing a protein expression construct into a host cell. Some expression vectors also replicate inside host cells, which increases protein expression by the protein expression construct. One type of vector is a "plasmid," which refers to a circular double stranded DNA loop into which additional DNA segments may be ligated. Other vectors include cosmids, bacterial artificial chromosomes (BAC) and yeast artificial chromosomes (YAC), fosmids, phage and phagemids. Another type of vector is a viral vector, wherein additional DNA segments may be ligated into the viral genome. Certain vectors are capable of autonomous replication in a host cell into which they are introduced (e.g., vectors having an origin of replication which functions in the host cell). Other vectors can be integrated into the genome of a host cell upon introduction into the host cell and are thereby replicated along with the host genome. Moreover, certain preferred vectors are capable of directing the expression of genes to which they are operatively linked.

Suitable bacterial cells include *Escherichia coli, Bacillus subtilis, Salmonella typhimurium, Pseudomonas* spp., *Streptomyces* spp., and *Staphylococcus* spp. Suitable yeast cells include *Saccharomyces* spp., *Pichia* spp., and *Kuyveromyces* spp. Suitable insect cells include those derived from *Bombyx mori, Mamestra brassicae, Spodoptera frugiperda, Trichoplusia ni* and *Drosophila melanogaster.* Such mammalian host cells include but are not limited to CHO, VERO, BHK, Hela, COS, MDCK, W138, BT483, Hs578T, HTB2, BT2O and T47D, NS0, CRL7O3O, HsS78Bst, human hepatocellular carcinoma cells (e.g. Hep G2), human adenovirus transformed 293 cells (e.g. HEK293), PER.C6, mouse L-929 cells, HaK hamster cell lines, murine 3T3 cells derived from Swiss, Balb-c or NIH mice, and CV-1 cell line cells.

The present invention also contemplates the use of wild type and recombinant antithrombin proteins that are conjugated or fused to any other protein, protein fragment, protein domain, peptide, small molecule or other chemical entity. For example, suitable fusion or conjugation partners include serum albumins (for example, bovine, rabbit or human), keyhole limpet hemocyanin, immunoglobulin molecules (including the Fc domain of immunoglobulins), thyroglobulin, ovalbumin, tetanus toxoid, a toxoid from other pathogenic bacteria, an attenuated toxin derivative, cytokines, chemokines, glucagon-like peptide-1, exendin-4, XTEN, or combinations thereof.

In one embodiment, the antithrombin having utility in the present invention may be fused to an immunoglobulin Fc domain. For example, the immunoglobulin Fc domain may comprise at least a portion of a constant heavy immunoglobulin domain. The constant heavy immunoglobulin domain is preferably an Fc fragment comprising the CH2 and CH3 domain and, optionally, at least a part of the hinge region. The immunoglobulin Fc domain may be an IgG, IgM, IgD, IgA or IgE immunoglobulin Fc domain, or a modified immunoglobulin Fc domain derived therefrom. Preferably, the immunoglobulin Fc domain comprises at least a portion of a constant IgG immunoglobulin Fc domain. The IgG immunoglobulin Fc domain may be selected from igG1, IgG2, IgG3 or IgG4 Fc domains, or modified Fc domains thereof.

It should be appreciated by those skilled in the art that the specific embodiments disclosed within paragraphs [0020] - [0031] should not be read in isolation, and that the present specification intends for these embodiments to be disclosed in combination with other embodiments as opposed to being disclosed individually. For example, where the context does not imply otherwise, each of the embodiments disclosed in paragraphs [0020] - [0031] is to be read as being explicitly combined with each of the embodiments in paragraphs [0010] - [0019], or any permutation of 2 or more of the embodiments disclosed therein.

### Dosing & Administration

In one embodiment, the pharmaceutical composition of the present invention comprising antithrombin could be delivered to the patient by an administration route selected from the group consisting of intravenous, subcutaneous, intramuscular, intradermal, intraperitoneal, intrapulmonary, intranasal, transdermal, transmucosal, oral, vaginal and rectal.

In certain embodiments, the pharmaceutical composition of the present invention comprising antithrombin is administered to the patient parenterally, for example intravenously or subcutaneously. In another embodiment, the pharmaceutical composition of the present invention comprising antithrombin is administered to the patient by an administration route selected from intrapulmonary or intranasal, for example using a nebuliser or inhaler device.

The antithrombin may be administered to the patient as part of a multiple-dosage regimen, wherein the antithrombin is administered to the patient using the same route of administration for each dose. In one embodiment, each dose of antithrombin is administered to the patient parenterally. For example, each dose of antithrombin may be administered to the patient intravenously.

The present invention also provides for a dosage regimen that incorporates combined modes of administration. For example, the initial dose may be administered parenterally, such as intravenously or subcutaneously, and subsequent doses may be delivered by an administration route selected from intrapulmonary or intranasal. In an alternative embodiment, the first 1 to 5 doses may be administered parenterally, such as intravenously or subcutaneously, and subsequent doses may be by an administration route selected from intrapulmonary or intranasal.

Alternatively, the initial dose may be delivered by an administration route selected from intrapulmonary or intranasal, and subsequent doses may be administered parenterally, such as intravenously or subcutaneously. In another embodiment, the first 1 to 5 doses may be delivered by an administration route selected from intrapulmonary or intranasal, and subsequent doses may be administered parenterally, such as intravenously or subcutaneously.

The multiple dosing period may comprise from about 5 to about 30 administrations up to a total cumulative dose. For example, the multiple dosing period may comprise from about 5 to about 25 administrations up to a total cumulative dose. In one embodiment, the multiple dosing period may comprise from about 5 to about 20 administrations up to a total cumulative dose. In another embodiment, the multiple dosing period may comprise from about 5 to about 15 administrations up to a total cumulative dose. In yet a further embodiment, the multiple dosing period may comprise from about 4 to about 20 administrations up to a total cumulative dose. In one embodiment, the multiple dosing period may comprise from about 2 to about 20 administrations up to a total cumulative dose. In another embodiment, the multiple dosing period may comprise from about 3 to about 10 administrations up to a total cumulative dose. In yet a further embodiment, the multiple dosing period may comprise from about 4 to about 10 administrations up to a total cumulative dose. In one embodiment, the multiple dosing period may comprise from about 5 to about 10 administrations up to a total cumulative dose.

The multiple dosing period may extend over a period of about 1 to about 30 weeks. For example, the multiple dosing period may extend over a period of about 1 to about 20 weeks. In one embodiment, the multiple dosing period may extend over a period of about 1 to about 10 weeks. In some embodiments, the multiple dosing period may extend over a period of about 1 to about 5 weeks. In one embodiment, the multiple dosing period may extend over a period of less than about 5 weeks. In yet a further embodiment, the multiple dosing period may extend over a period of less than about 3 weeks.

The multiple portion doses may be administered at equally spaced intervals of from about 1 day to about 30 days. For example, the multiple portion doses may be administered at equally spaced intervals of from about 1 day to about 20 days. In some embodiments, the multiple portion doses may be administered at equally spaced intervals of from about 1 day to about 15 days. In one embodiment, the multiple portion doses may be administered at equally spaced intervals of from about 1 day to about 10 days. In a further embodiment, the multiple portion doses may be administered at equally spaced intervals of from about 1 day to about 7 days.

Alternatively, the multiple portion doses may be administered at unequal intervals of from about 1 day to about 30 days. For example, the multiple portion doses may be administered at unequally spaced intervals of from about 1 day to about 20 days. In some embodiments, the multiple portion doses may be administered at unequally spaced intervals of from about 1 day to about 15 days. In one embodiment, the multiple portion doses may be administered at unequally spaced intervals of from about 1 day to about 10 days. In a further embodiment, the multiple portion doses may be administered at unequally spaced intervals of from about 1 day to about 7 days.

As used herein, the term "unequally spaced intervals" shall mean that at least one of the multiple portion doses is administered at a different interval to the other doses. For example, the term shall be construed to include a dosing regimen in which the first 5 doses are administered at an interval of 1 day, and the subsequent 5 doses are administered at an interval of 7 days.

It should be appreciated by those skilled in the art that the specific embodiments disclosed within paragraphs [0033] - [0042] should not be read in isolation, and that the present specification intends for these embodiments to be disclosed in combination with other embodiments as opposed to being disclosed individually. For example, where the context does not imply otherwise, each of the embodiments disclosed in paragraphs [0033] - [0042] is to be read as being explicitly combined with each of the embodiments in paragraphs [0010] - [0032], or any permutation of 2 or more of the embodiments disclosed therein.

### Pharmaceutical Compositions of the Invention

The pharmaceutical compositions of the present invention may optionally further comprise at least one pharmaceutically acceptable carrier. The at least one pharmaceutically acceptable carrier may be chosen from adjuvants and vehicles. The at least one pharmaceutically acceptable carrier, includes any and all solvents, diluents, other liquid vehicles, dispersion aids, suspension aids, surface active agents, isotonic agents, thickening agents, emulsifying agents, and preservatives as suited to the particular dosage form desired.

Suitable carriers are described in Remington: The Science and Practice of Pharmacy, 21 st edition, 2005, ed. D.B. Troy, Lippincott Williams & Wilkins*,* Philadelphia, and Encyclopedia of Pharmaceutical Technology, eds. J. Swarbrick and J. C. Boylan, 1988-1999, Marcel Dekker, New York*,* the contents of which are incorporated herein by reference. Preferred examples of such carriers or diluents include, but are not limited to, water, saline, Ringer's solutions, glycols, dextrose solution, buffered solutions (such as phosphates, glycine, sorbic acid, and potassium sorbate) and human serum albumin. Liposomes and non-aqueous vehicles such as glyceride mixtures of saturated vegetable fatty acids, and fixed oils (such as peanut oil, cottonseed oil, safflower oil, sesame oil, olive oil, com oil and soybean oil) may also be used depending on the route of administration.

The pharmaceutical composition of the invention is formulated to be compatible with its intended route of administration. For systemic use, the pharmaceutical composition of the invention can be formulated for administration by a conventional route selected from the group consisting of intravenous, subcutaneous, intramuscular, intradermal, intraperitoneal, intracerebral, intracranial, intrapulmonary, intranasal, intraspinal, intrathecal, transdermal, transmucosal, oral, vaginal, and rectal.

In one embodiment, parenteral administration is the chosen route of administration. The pharmaceutical composition may be enclosed in ampoules, disposable syringes, sealed bags, or multiple dose vials made of glass or plastic. In one embodiment, administration as an intravenous injection is the chosen route of administration. The formulations can be administered continuously by infusion or by bolus injection.

Pharmaceutical compositions suitable for injectable use include sterile aqueous solutions (where water soluble) or dispersions, and sterile powders for the extemporaneous preparation of sterile injectable solutions or dispersions. For intravenous administration, suitable carriers include physiological saline, bacteriostatic water, CREMOPHOR EL, or phosphate buffered saline (PBS). In some embodiments, the carrier may be a solvent or dispersion medium containing, for example water, ethanol, polyols (for example, glycerol, propylene glycol, and liquid polyethylene glycol, and the like), and suitable mixtures thereof. In all cases, the composition must be sterile and should be fluid to the extent that easy syringe-ability exists.

Prevention of the growth of microorganisms can be achieved by various antibacterial and antifungal agents, for example parabens, chlorobutanol, phenol, ascorbic acid, thimerosal, and the like. In many cases, it will be preferable to include isotonic agents, for example sugars (such as mannitol, sorbitol, etc.), polyalcohols, or sodium chloride in the composition. In some embodiments, the compositions of the present invention may include stabilising agents such as amino acids; for example alanine, glycine, and combinations thereof. Prolonged absorption of the injectable compositions can be brought about by including in the composition an agent which delays absorption, for example aluminum monostearate or gelatin.

Sterile injectable solutions of the pharmaceutical composition of the present invention can be prepared by incorporating the active molecule in the required amount in an appropriate solvent with one or a combination of ingredients as discussed above followed by filtered sterilization. In the case of sterile powders for the preparation of sterile injectable solutions, methods of preparation include vacuum drying and freeze-drying that provide a powder of the active ingredient plus any additional desired ingredient from a previously sterile-filtered solution thereof.

In one embodiment, the antithrombin may constitute at least 20 % by weight of the total protein content of the injectable pharmaceutical composition of the present invention. For example, the antithrombin may constitute greater than or equal to about 30 %, 40 %, 50 %, 60 %, 70 %, 75 %, 80 %, 85 %, 90 %, 93 %, 95 %, 96 %, 97 %, 98 %, or 99 % by weight of the total protein content of the injectable pharmaceutical composition of the present invention.

The injectable pharmaceutical compositions of the present invention may be presented as a unit dosage unit form, *i.e.* as physically discrete units intended as unitary dosages for the subject to be treated.

In one embodiment, the present invention provides for aerosolized administration of antithrombin. The aerosol may be a suspension of fine solid particles or liquid droplets in any pharmaceutically acceptable propellant gas. In one embodiment, the antithrombin may be formulated as a dry powder suitable for use or atomisation in an inhalation device.

It should be appreciated by those skilled in the art that the specific embodiments disclosed within paragraphs [0044] - [0053] should not be read in isolation, and that the present specification intends for these embodiments to be disclosed in combination with other embodiments as opposed to being disclosed individually. For example, where the context does not imply otherwise, each of the embodiments disclosed in paragraphs [0044] - [0053] is to be read as being explicitly combined with each of the embodiments in paragraphs [0010] - [0043], or any permutation of 2 or more of the embodiments disclosed therein.

### Combination Therapy

The present invention also contemplates the use of supplementary active compounds and molecules in combination with antithrombin. The supplementary active compounds and molecules can be co-formulated with antithrombin as a unit dosage form, *i.e.* as a physically discrete unit intended as a unitary dosage for the subject to be treated.

Alternatively, the supplementary active compounds and molecules can be presented as a kit-of-parts, and:
- administered separately to antithrombin, in a phased or sequential dosing pattern; or
- co-administered simultaneously from different dosage forms.

For example, the present invention contemplates administering other serum, or plasma-based proteins in combination with antithrombin. Serum or plasma proteins within the scope of the present invention include those purified from a suitable plasma source, such as human plasma, and those prepared using recombinant manufacturing techniques. For example, the serum or plasma protein may be selected from the group consisting of albumin (*e.g*. ALBUTEIN), alpha-1 antitrypsin (*e.g*. PROLASTIN), polyclonal immunoglobulins (IgG, IgA, and combinations thereof), polyspecific immunoglobulins (IgM), C1 esterase inhibitor (*e.g*. BERINERT), transthyretin, transferrin, lactoferrin, and combinations thereof.

Exemplary polyclonal immunoglobulins within the scope of the present invention include commercially available polyclonal IgG formulations such as FLEBOGAMMA DIF 5 % & 10 %, GAMUNEX- C 10 %, BIVIGAM 10 %, GAMMAGARD Liquid 10 %, *etc.*

Exemplary polyspecific immunoglobulins (IgM) within the scope of the present invention include commercially available immunoglobulin formulations containing polyspecific IgM such as PENTAGLOBIN or TRIMODULIN.

In one embodiment, the serum or plasma protein may be selected from the group consisting of albumin, alpha-1 antitrypsin, transferrin, lactoferrin, and combinations thereof. For example, the serum or plasma protein may be selected from the group consisting of alpha-1 antitrypsin, transferrin, lactoferrin, and combinations thereof.

In a particular embodiment, a therapeutically effective amount of protein selected from transferrin, lactoferrin, and combinations thereof is administered to the patient in addition to the antithrombin. In one embodiment, a therapeutically effective amount of transferrin is administered to the patient in addition to antithrombin.

As used herein, the term "transferrin" shall include transferrin at all iron saturation levels including the commonly termed holo-transferrin and apo-transferrin forms. By "apo-transferrin" it is meant transferrin having an iron saturation of less than about 5 %. By "holo-transferrin" it is meant transferrin having an iron saturation of about 95 % or greater.

Similarly, as used herein, the term "lactoferrin" shall include lactoferrin at all iron saturation levels including the commonly termed holo-lactoferrin and apo-lactoferrin forms. By "apo-lactoferrin" it is meant lactoferrin having an iron saturation of less than about 5 %. By "holo-lactoferrin" it is meant lactoferrin having an iron saturation of about 95 % or greater.

In one embodiment, with respect to the combination therapy of the present invention, the antithrombin may constitute at least 20 % by weight of the total protein content utilised in the combination therapy of the present invention. For example, the antithrombin may constitute greater than or equal to about 30 %, 40 %, 50 %, 60 %, 70 %, 75 %, 80 %, 85 %, 90 %, 93 %, 95 %, 96 %, 97 %, 98 %, or 99 % by weight of the total protein content utilised in the combination therapy of the present invention.

It should be appreciated by those skilled in the art that the specific embodiments disclosed within paragraphs [0055] - [0064] should not be read in isolation, and that the present specification intends for these embodiments to be disclosed in combination with other embodiments as opposed to being disclosed individually. For example, where the context does not imply otherwise, each of the embodiments disclosed in paragraphs [0055] - [0064] is to be read as being explicitly combined with each of the embodiments in paragraphs [0010] - [0054], or any permutation of 2 or more of the embodiments disclosed therein.

### Brief Description of the Drawings

Additional features and advantages of the present invention will be made clearer in the appended drawings, in which:
**Figures 1-4** illustrate the effect of varying doses of antithrombin administered *via* the intraperitoneal route in a middle cerebral artery occlusion mouse model; and
**Figure 5** correlates the doses of antithrombin administered by the intraperitoneal route to plasmatic antithrombin activity, and the resulting beneficial effect on the variables measured in Figures 1-4.

### Detailed Examples of the Invention

It should be readily apparent to one of ordinary skill in the art that the examples disclosed herein below represent generalised examples only, and that other arrangements and methods capable of reproducing the invention are possible and are embraced by the present invention.

### Example 1: Evaluation of the Effect of Antithrombin III (ATIII) in a Middle Cerebral Artery Occlusion Mouse Model

C57/BL6 mice at 8-10 weeks of age and approximately 20 g in weight, were placed under isoflurane anesthesia, and an occlusion of the left common carotid artery (CCA) was performed. A permanent suture around the external common carotid artery (ECA) was subsequently performed. Then, a 6.0 silicon-coated monofilament suture was introduced into the ECA. The occluder is introduced to occlude the origin of the middle cerebral artery (MCA). The occlusion is carried out for 60 minutes. During the procedure, temperature control of the mice was in operation. After that (within two hours after occlusion), the animals were assessed by neuroscore analysis. The cut-off used in the study was a neuroscore ≥ 4.

5 different groups were assessed: sham, MCAO, MCAO + ATIII (incorporating 3 groups with different ATIII doses). In groups with MCAO mice, initially n=8 animals/group are included in the study. Sham group is n=5 animals. For appropriate controls, sham and MCAO mice were administered with the same volume of excipient solution and ATIII respectively. The different ATIII treatment groups were administered with the same volume of appropriate dilutions of ATIII.

At 3 hours post-MCAO, varying doses of ATIII were intraperitoneally administered to mice (250, 500 and 750 IU/Kg). This administration was subsequently repeated at 24h and 48h post-MCAO. At 54 hours post-MCAO (sacrifice), mice were assessed by neuroscore analysis, a plasma sample collected, and the brains were perfused.

Plasma samples were obtained by cardiac puncture at sacrifice for the analysis of plasmatic ATIII activity. **Table 1** illustrates the 7-point neuroscore scale used to evaluate the degree of the brain lesion after MCAO.

**Table 1**

| Score | |
|---|---|
| 0 | No observable deficit |
| 1 | Failure to extend the contralateral forepaw |
| 2 | Mild circling behavior when picked up by the tail. <50 % attempts to rotate to the contralateral side |
| 3 | Mild consistent circling. >50 % attempts to rotate to the contralateral side |
| 4 | Consistent and strong circling, the mouse holds a rotation position for more than 1 or 2 seconds, with its nose almost reaching its tail |
| 5 | Severe rotation with failing in a direction contralateral to the infarct, loss of walking or righting reflex |
| 6 | Depressed level of consciousness, comatose or moribund |

Brains were prepared for analysis as follows. Mice were transcardially perfused with ice cold heparinized (Heparin 2.5 IU/ml) saline in order to remove blood from the brains. Fresh brains were removed, and the whole left hemisphere block were placed in 4 % paraformaldehyde in 0.1 M phosphate buffer (PB) for 24 hours at +4°C, cryoprotected in 30 % sucrose in 0.1 M PB for 2-3 days on a shaker at +4°C. The brain was then dried, placed on top of a vial cork and frozen on top of liquid nitrogen, and the block was then stored at -80°C until cryostat sections are obtained. Lesion volume was measured by MAP2 staining. To quantify apoptotic cell death, immunofluorescence against cleaved caspase-3 (Cell Signaling Technology) was measured. To study the neutrophil infiltration, immunofluorescence using the anti-neutrophil antibody NIMP-R14 (Abcam). In both cases, quantification was performed using stereological analysis.

The neuroscore evaluation post MCAO establishes whether MCAO-mediated brain lesion was induced within the mice; no differences in neuroscore value existed between groups before ATIII treatment. Four animals showed no neuroscore deficit (cut-off>4) and the final distribution was:
Sham - 5 animals;
MCAO - 7 animals;
MCAO + 250 IU/Kg ATIII - 7 animals;
MCAO + 500 IU/Kg ATIII - 7 animals;
MCAO + 750 IU/Kg ATIII - 7 animals.

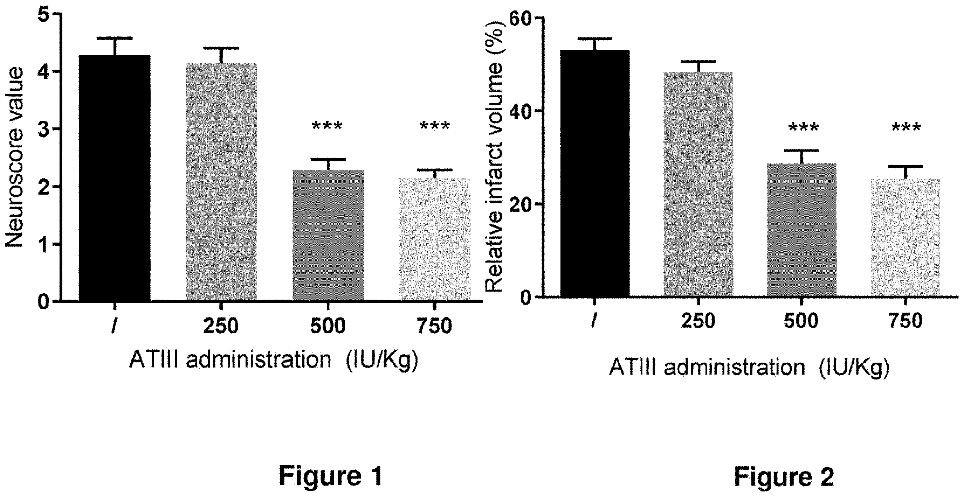

The neuroscore was evaluated in a blinded manner at 54 hours. The results are presented in **Figure 1****.** Groups were compared using ANOVA with Bonferroni post hoc analysis (***p<0.001 vs. MCAO-excipient group). From **Figure 1** it is evident that the MCAO + 250 IU/Kg group showed no differences compared to MCAO group (MCAO: 4.29±0.31; MCAO+250 ATIII: 4.14±0.28). Interestingly, the administration of 500 and 750 IU/Kg of ATIII resulted in a statistically significant improved neuroscore value compared to MCAO group (MCAO+500 A Till: 2.29±0.18; MCAO+750 ATIII: 2.14±0.15). The sham group showed a neuroscore value of 0.

The lesion size/volume was measured in the different groups. The results are presented in **Figure 2****.** Groups were compared using ANOVA with Bonferroni post hoc analysis (***p<0.001 vs. MCAO-excipient group). The lesion volume was expressed as a % of the relative infarct volume. From **Figure 2** we see that the MCAO group showed a 53±2.5 % volume lesion size. The administration of ATIII resulted in a statistically significant reduction of the MCAO-induced volume lesion (MCAO + 500 ATIII: 29±2.8 %; MCAO + 750 ATIII: 25±2.8 %). In contrast, the administration of 250 IU/Kg of ATIII did not decrease the volume of the lesion (MCAO + 250 ATIII: 48±2.4 %).

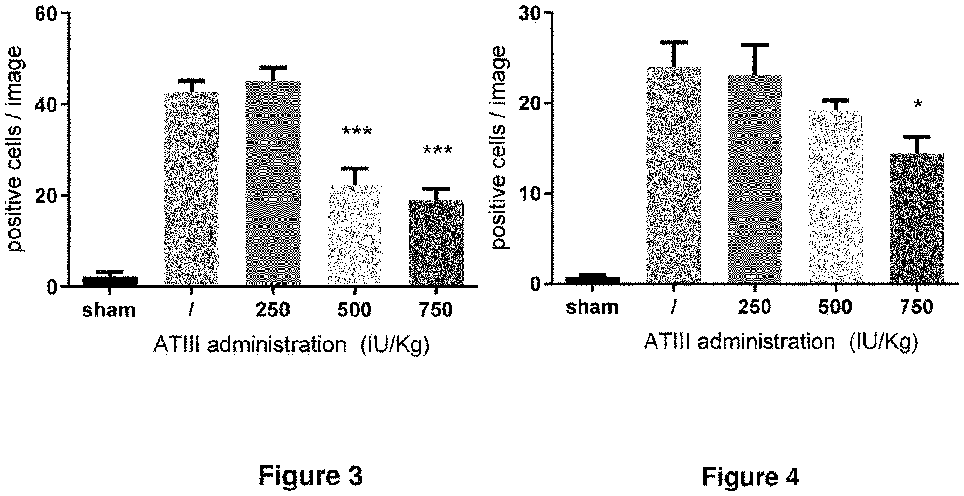

The effect of ATIII on the levels of caspase-3 is illustrated in **Figure 3****.** Groups were compared using ANOVA with Bonferroni post hoc analysis (P values ***p<0.001 vs MCAO-excipient). MCAO induced lesion resulted in a large increase of caspase-3 activation. However, administration of ATIII significantly reduced the activation of caspase-3 at 500 and 750 IU/Kg. No differences between MCAO group and MCAO + ATIII 250 mg/ Kg were detected. Thus, ATIII may mediate its effects on neurological amelioration and volume lesion reduction *via* an antiapoptotic effect.

The effect of ATIII on neutrophil infiltration is illustrated in **Figure 4****.** Groups were compared using ANOVA with Bonferroni post hoc analysis (P values *p<0.05 vs. MCAO-excipient). MCAO induced lesion caused an increase in neutrophil infiltration. The administration of ATIII significantly reduced the neutrophil infiltration solely at a dose of 750 lU/Kg. No differences between MCAO group and MCAO + ATIII 250 and 500 IU/Kg were detected. Thus, ATIII may mediate its effects on neurological amelioration and volume lesion reduction *via* the preservation of blood brain barrier permeability.

### Example 2: Correlation of Antithrombin III (ATIII) administered by the Intraperitoneal Route to Plasmatic ATIII Activity

Plasma samples were obtained by cardiac puncture at sacrifice for the analysis of plasmatic ATIII activity. The correlation of plasmatic ATIII activity for each intraperitoneal (IP) dose administered is tabulated in **Table 2,** *infra.*

**Table 2**

| **IP Dose** | **Plasmatic ATIII activity** |
|---|---|
| 250 IU/Kg | 110% - 130% |
| 500 IU/Kg | 130% - 160% |
| 750 IU/Kg | 125 % - 145 % |

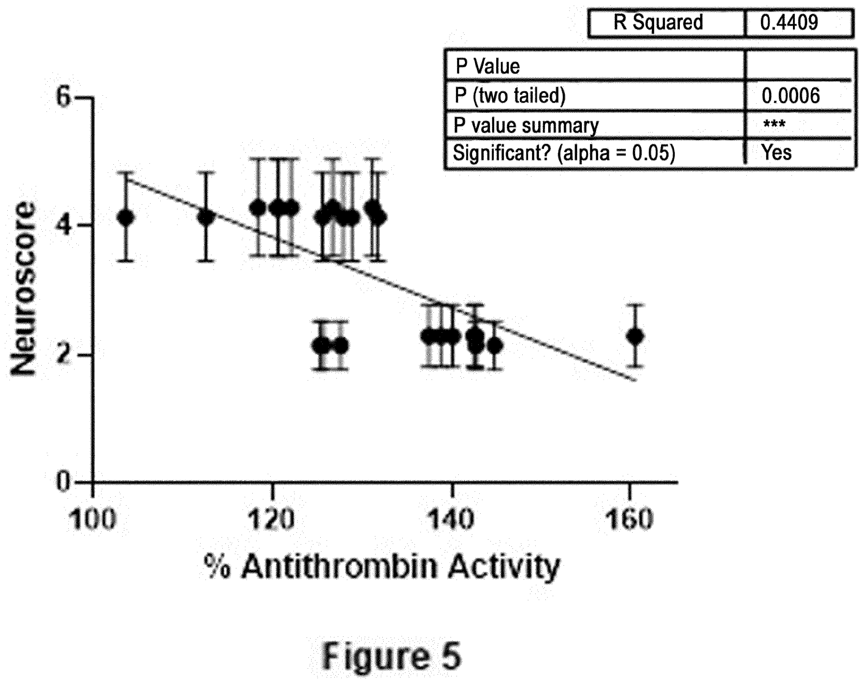

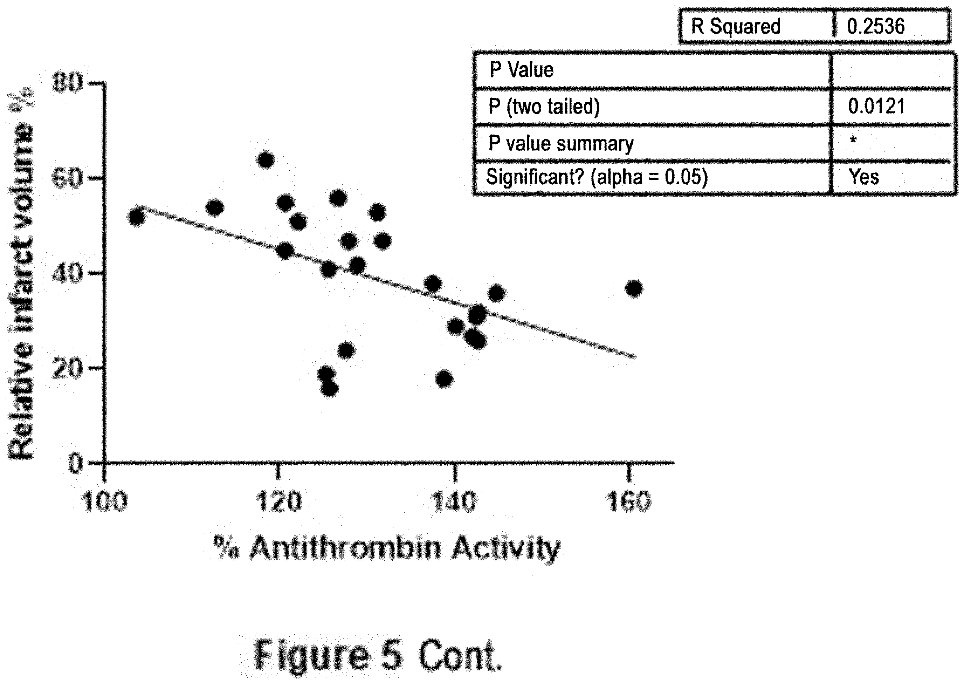

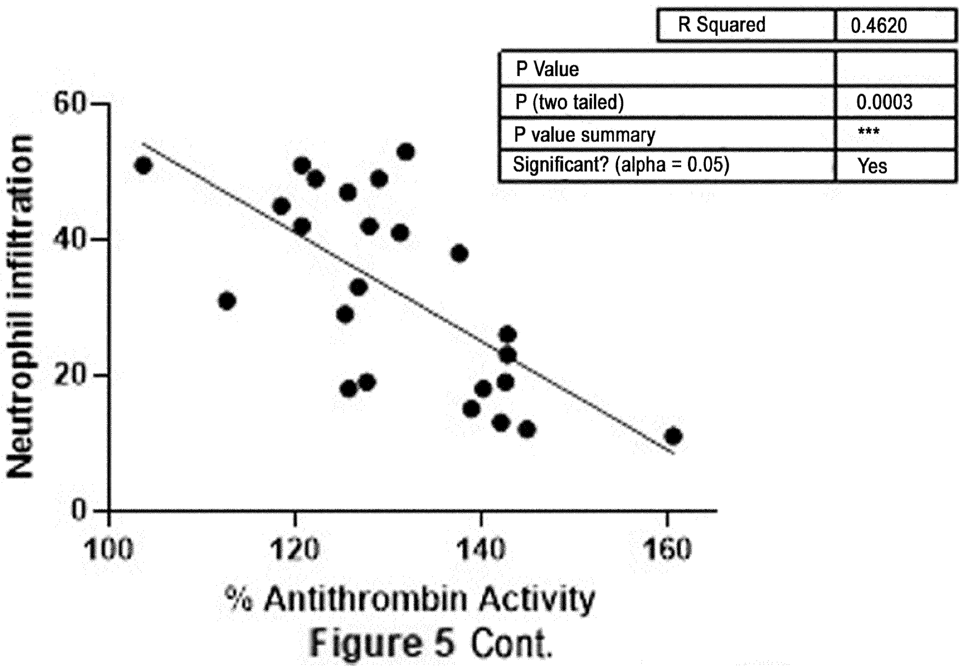

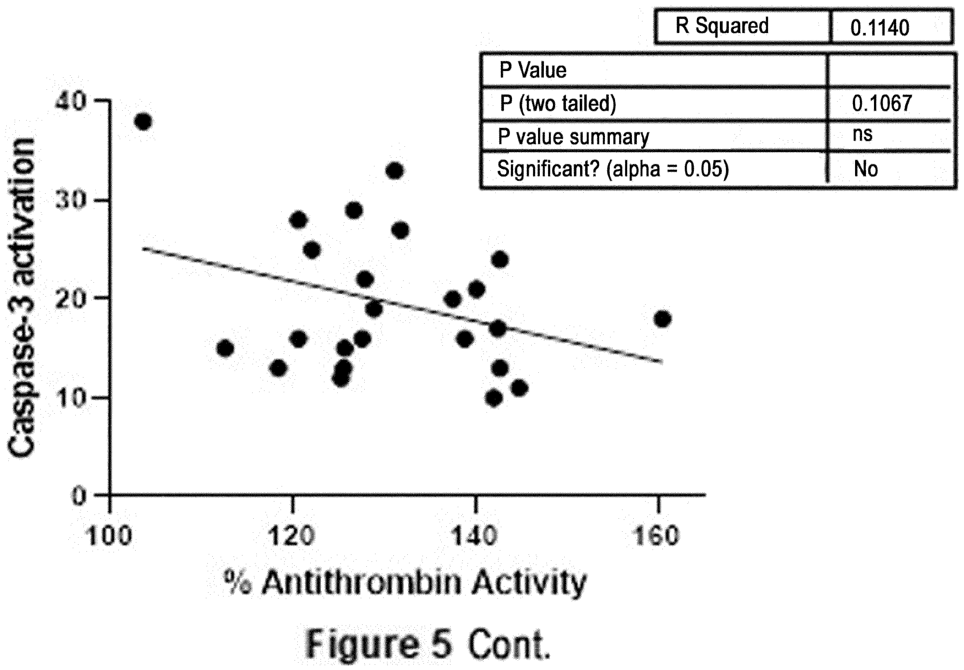

**Figure 5** re-plots each of the bio-variables measured in **Figures 1-4** as a function of plasmatic ATIII activity. It is abundantly clear from the figures that increasing plasmatic ATIII activity has a beneficial effect in ameliorating the effects of the MCAO mouse procedure.

### Sequences

The sequences referred to in the preceding text are outlined below in fasta format. In the event of a discrepancy between the sequence listed in this text and the corresponding sequence in the accompanying sequence listing, the sequence listed in this text shall be the prevailing sequence for the purposes of correcting an error.

### SEQ ID No. 1 Antithrombin

## Claims

1. A pharmaceutical composition comprising antithrombin for use in the treatment of a stroke, wherein the antithrombin is administered at a dose sufficient to:
increase the patient's plasmatic antithrombin activity to greater than about 120 %; or
increase the patient's plasmatic antithrombin concentration to greater than about 1.2 IU/mL.

2. The composition claim 1 wherein the stroke is selected from the group consisting of an ischemic stroke and a focal brain ischemic stroke.

3. The composition of claim 1 or 2 wherein the antithrombin is administered at a dose sufficient to increase the patient's plasmatic antithrombin activity to greater than about 120 % but less than about 200 %.

4. The composition of claim 1 or 2 wherein the antithrombin is administered at a dose sufficient to increase the patient's plasmatic antithrombin concentration to greater than about 1.2 lU/mL but less than about 2.0 IU/mL.

5. The composition of claim 1 or 2 wherein the antithrombin is administered at a dose sufficient to increase the patient's plasmatic antithrombin levels to greater than about 140 µg/mL but less than about 267 µg/mL.

6. The composition of any preceding claim wherein the antithrombin is administered in multiple doses as part of a multiple-dosage regimen.

7. The composition of claim 6 wherein the antithrombin is administered at a dose sufficient to increase the patient's plasmatic antithrombin activity to greater than about 120 % at at least one time point in a multiple dosage time period.

8. The composition of claim 6 wherein the antithrombin is administered such that the patient's plasmatic antithrombin concentration increases to greater than about 1.2 IU/mL at at least one time point in a multiple dosage time period.

9. The composition of claim 6 wherein the antithrombin is administered such that the patient's plasmatic antithrombin levels increase to greater than about 140 µg/mL at at least one time point in a multiple dosage time period.

10. The composition of claims 6 to 9 wherein the multiple dosing regimen comprises from about 2 to about 30 administrations up to a total cumulative dose.

11. The composition of claim 10 wherein the multiple dosing regimen comprises from about 5 to about 20 administrations up to a total cumulative dose.

12. The composition of claims 6 to 11 wherein the multiple dosage regimen extends over a period of about 1 to about 30 weeks.

13. The composition of claims 6 to 12 wherein the multiple dosing regimen extends over a period of about 1 to about 10 weeks.

14. The composition of claims 6 to 13 wherein the multiple dosing regimen comprises multiple portion doses administered at equally spaced intervals of from about 1 day to about 30 days.

15. The composition of claims 6 to 13 wherein the multiple dosing regimen comprises multiple portion doses administered at equally spaced intervals of from about 1 day to about 10 days.

16. The composition of claims 6 to 13 wherein the multiple dosing regimen comprises multiple portion doses administered at unequally spaced intervals of from about 1 day to about 30 days.

17. The composition of claims 6 to 13 wherein the multiple dosing regimen comprises multiple portion doses administered at unequally spaced intervals of from about 1 day to about 10 days.

18. The composition of any preceding claim wherein the antithrombin is administered by an administration route selected from the group consisting of intravenous, subcutaneous, intramuscular, intradermal, intraperitoneal, intrapulmonary, intranasal, and combinations thereof.

19. The composition of any preceding claim wherein the antithrombin is administered as part of a combination therapy with at least one other plasma protein selected from the group consisting of: albumin, alpha-1 antitrypsin, transferrin, lactoferrin, polyclonal immunoglobulins, polyspecific immunoglobulins, C1 esterase inhibitor, transthyretin, and combinations thereof.

20. The composition of claim 19 wherein the antithrombin is administered as part of a combination therapy with transferrin.

21. The composition of claim 20 wherein the transferrin has an iron saturation of about 30 % or less.

22. The composition claims 19 to 21 wherein the antithrombin and the plasma protein are administered as a unitary dosage form.
